# EUROPEAN PATENT APPLICATION

(11) **EP 4 834 341 A1**
(43) Date of publication of application: **04.11.2026**
(21) Application number: 24911164.2
(22) Date of filing: 25.12.2024
(51) Int. Cl.: A61K 31/352, A61P 27/02

(54) **PHARMACEUTICAL COMPOSITION AND METHOD FOR TREATING AND/OR PREVENTING CORNEAL INJURY**

(30) Priority: 25.12.2023 CN 202311793641
(71) Applicant: Suzhou Glenkol Pharma Technology Co., Ltd., Suzhou, Jiangsu, 215127 (CN); Quanzhou Haichuang Pharmaceutical Technology Co., Ltd., Quanzhou, Fujian 362221 (CN); Huiankai (Xiamen) Pharmaceutical Technology Co., Ltd., Xiamen, Fujian 361028 (CN)
(72) Inventor: CHEN, Jinhui, Suzhou, Jiangsu 215127 (CN); WANG, Xiaoting, Suzhou, Jiangsu 215127 (CN)
(74) Representative: Hautier IP - MC/EP
(86) International application number: PCT/CN2024/142095
(87) International publication number: WO 2025/140240

(57) **Abstract**

The present disclosure provides use of 7,8-dihydroxyflavone in treating corneal injury. Specifically, the present disclosure provides use of 7,8-dihydroxyflavone in preparing a medicament for treating corneal injury.

## Description

The present disclosure claims the priority to Chinese Patent Application CN202311793641.2 filed on December 25, 2023, the disclosure of which is hereby incorporated by reference in its entirety.

### TECHNICAL FIELD

The present disclosure relates to the field of biomedical technology, specifically to a method and a pharmaceutical composition for treating and/or preventing corneal injury.

### BACKGROUND

The eyes are known as the "windows to the soul." Eye health concerns people of all ages throughout their entire lifespans and is a major public health issue concerning the well-being of the population. The cornea is the transparent tissue covering the front of the eyeball, containing abundant corneal nerves and being one of the most sensitive tissues in the body. Corneal nerves play a crucial role in sensing temperature, chemical stimuli, mechanical stimuli, and pain. They function to nourish corneal epithelial cells and corneal stromal cells, maintain the health of the ocular surface and corneal sensitivity, and play a vital role in protecting the integrity of the corneal epithelium, promoting the proliferation and migration of corneal epithelial and stromal cells, as well as facilitating the repair of corneal injury. Corneal injury is a common condition in ophthalmic practice, primarily including traumatic injuries (such as corneal chemical burn, corneal mechanical injury, ophthalmic surgical injury, and biological injury), infectious injury, inflammatory injury, and injury caused by tear film dysfunction (including injuries to the corneal epithelium and corneal nerves resulting from tear deficiency, abnormal tear dynamics, abnormal mucin secretion, or abnormal lipid secretion, which also constitute the primary etiology of dry eye disease). Corneal nerve injury resulting from ocular trauma is also a primary cause of neurotrophic keratitis. Delayed treatment may lead to corneal epithelial defect, corneal opacity, and corneal neovascularization, further progressing to corneal ulceration or corneal melting, and even result in corneal perforation. This severely impairs visual function and leaves patients with lifelong pain and suffering.

The etiologies of corneal injury, neurotrophic keratitis, and dry eye disease are complex and diverse. Current treatment methods for corneal injury primarily include: (1) administering medications for promoting corneal epithelial repair, such as artificial tears that help stabilize the tear film and protect the corneal epithelium, as well as therapeutic agents including sodium hyaluronate eye drops, deproteinized calf blood extract eye drops or gels, and 20%-100% autologous serum therapy; (2) for patients with ocular surface inflammatory reaction, concurrent administration of low-concentration corticosteroids or immunosuppressants for anti-inflammatory treatment, such as 0.02% fluorometholone eye drops or 0.05% cyclosporine A eye drops. However, clinically available therapeutic drugs remain quite limited, and ocular symptoms in many patients (such as eye redness, ocular stinging pain, photophobia and epiphora, tearing, foreign body sensation, and dryness) fail to be alleviated, resulting in severe visual impairment.

7,8-Dihydroxyflavone (abbreviated: 7,8-DHF, CAS No.: 38183-03-8) is a flavonoid derivative. To date, there have been no reports on the application of 7,8-dihydroxyflavone in the treatment of corneal injury.

### SUMMARY

The present disclosure aims to address, at least to a certain extent, one of the technical problems existing in the prior art. To this end, the present disclosure provides a method for treating and/or preventing corneal injury, neurotrophic keratitis, or dry eye disease by using 7,8-dihydroxyflavone or a pharmaceutically acceptable salt thereof.

In a first aspect of the present disclosure, provided is use of 7,8-dihydroxyflavone or a pharmaceutically acceptable salt thereof in the manufacture of a medicament for treating and/or preventing corneal injury, neurotrophic keratitis, or dry eye disease.

In a second aspect of the present disclosure, provided is a pharmaceutical composition for preventing and/or treating corneal injury, neurotrophic keratitis, or dry eye disease, wherein the pharmaceutical composition includes 7,8-dihydroxyflavone or a pharmaceutically acceptable salt thereof, together with pharmaceutically acceptable excipients.

In some embodiments, the pharmaceutical composition is an oral formulation, an injectable formulation, or an ophthalmic formulation; wherein the oral formulation may be selected from a tablet, a capsule , a granule, a powder, an oral solution, or an oral suspension; the injectable formulation may be selected from an injection, a powders for injection, or a concentrated solution for injection; and the ophthalmic formulation may be selected from a patch, a lotion, a wash, a douche, an irrigation, an eye drop, an eye ointment, an ophthalmic gel, a solution for intraocular injection, a fumigant, or an eye mask.

In a third aspect of the present disclosure, provided is a method for preventing and/or treating corneal injury, neurotrophic keratitis, or dry eye disease, including administering to a subject 7,8-dihydroxyflavone or a pharmaceutically acceptable salt thereof, or the pharmaceutical composition according to the second aspect.

In some embodiments, the neurotrophic keratitis or dry eye disease is caused by corneal injury.

In some embodiments, the corneal injury is corneal epithelial injury and/or corneal nerve injury.

In some embodiments, the corneal nerve injury is selected from corneal intraepithelial nerve terminal injury, and/or corneal subbasal nerve plexus injury, and/or corneal stromal nerve trunk injury.

In some embodiments, the corneal injury may be a corneal chemical burn, a corneal mechanical injury, a corneal foreign body injury, a corneal biological injury, or a corneal thermal burn; alternatively, the corneal injury caused by tear film dysfunction, corneal infection, corneal inflammation, ciliary nerve injury, injury to the ophthalmic branch of the trigeminal nerve, neurotrophic keratitis, dry eye disease, or ophthalmic surgery.

In some embodiments, the effective amount of 7,8-dihydroxyflavone ranges from 0.1 to 1000 mg/kg body weight, for example 1 to 100 mg/kg body weight, such as 50 to 100 mg/kg body weight.

### BRIEF DESCRIPTION OF DRAWINGS

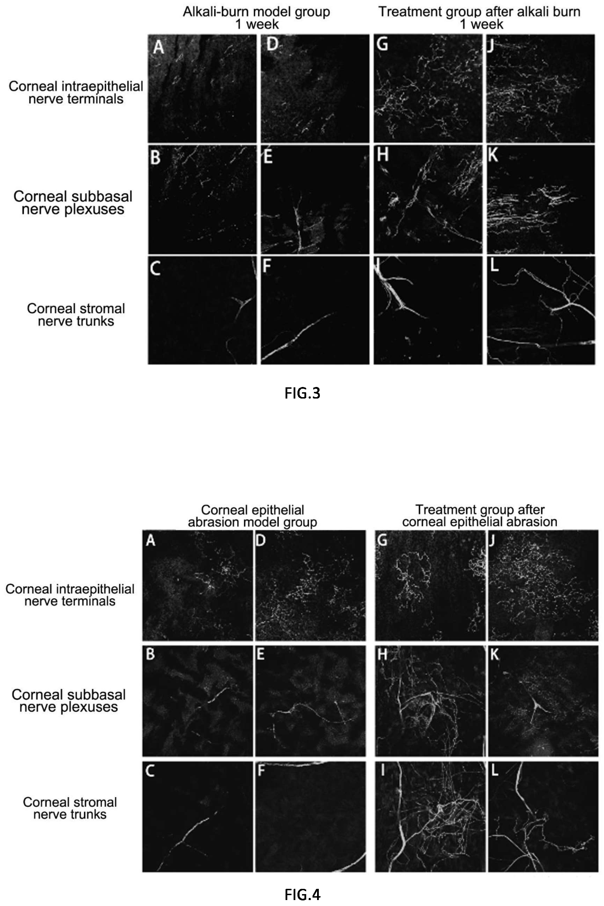

FIG. 1 shows a statistical comparison of corneal fluorescein staining scores among mice in the 7,8-DHF treatment group, model group, and normal control group according to Example 1.
FIG. 2 shows a comparison of tear secretion measurement results among mice in the 7,8-DHF treatment group, model group, and normal control group according to Example 1.
FIG. 3 shows a comparison of corneal nerve staining signals in mice treated with 7,8-DHF following corneal injury induced by alkali burn according to Example 2, wherein:
   (A) and (D) show staining signals of corneal intraepithelial nerve terminals in mice of corneal alkali-burn model group one week post injury;
   (B) and (E) show staining signals of corneal subbasal nerve plexuses in mice of corneal alkali-burn model group one week post injury;
   (C) and (F) show staining signals of corneal stromal nerve trunks in mice of corneal alkali-burn model group one week post injury;
   (G) and (J) show staining signals of corneal intraepithelial nerve terminals in mice treated with 7,8-DHF for one week following corneal alkali-burn;
   (H) and (K) show staining signals of corneal subbasal nerve plexuses in mice treated with 7,8-DHF for one week following corneal alkali-burn; and
   (I) and (L) show staining signals of corneal stromal nerve trunks in mice treated with 7,8-DHF for one week following corneal alkali-burn.
   As shown in FIG. 3, following corneal alkali-burn in mice, one-week 7,8-DHF treatment significantly ameliorated corneal intraepithelial nerve terminal injury, corneal subbasal nerve plexuses injury and corneal stromal nerve trunk injury, and significantly elevated corneal nerve staining signal density compared with untreated model group mice.
FIG. 4 shows a comparison of corneal nerve staining signals in mice treated with 7,8-DHF following corneal injury induced by epithelial abrasion according to Example 3, wherein:
   (A) and (D) show staining signals of corneal intraepithelial nerve terminals in mice of the corneal epithelial abrasion model group at one week post injury;
   (B) and (E) show staining signals of corneal subbasal nerve plexuses in mice of the corneal epithelial abrasion model group at one week post injury;
   (C) and (F) show staining signals of corneal stromal nerve trunks in mice of the corneal epithelial abrasion model group at one week post injury;
   (G) and (J) show staining signals of corneal intraepithelial nerve terminals in mice treated with 7,8-DHF for one week following corneal epithelial abrasion;
   (H) and (K) show staining signals of corneal subbasal nerve plexuses in mice treated with 7,8-DHF for one week following corneal epithelial abrasion; and
   (I) and (L) show staining signals of corneal stromal nerve trunks in mice treated with 7,8-DHF for one week following corneal epithelial abrasion.
   As shown in FIG. 4, following corneal epithelial abrasion in mice, one-week treatment with 7,8-DHF significantly ameliorated injuries to corneal intraepithelial nerve terminals, corneal subbasal nerve plexuses and corneal stromal nerve trunks, and significantly increased the density of corneal nerve staining signals compared with untreated corneal epithelial abrasion model group mice.

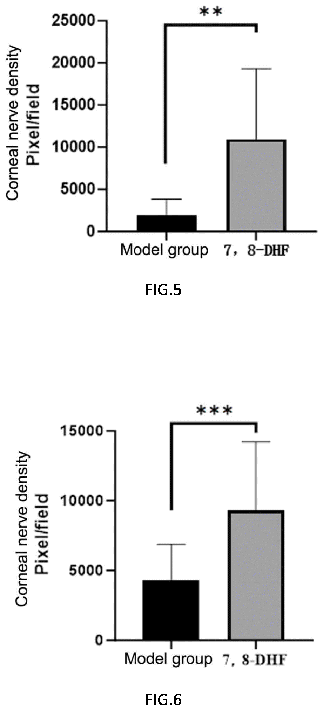

FIG. 5 shows a statistical comparison of corneal nerve density in mice between the 7,8-DHF treatment group and the model group according to Example 2.
FIG. 6 shows a statistical comparison of corneal nerve density in mice between the 7,8-DHF treatment group and the model group according to Example 3.

### DETAILED DESCRIPTION

The present disclosure provides a pharmaceutical composition for preventing and/or treating corneal injury, neurotrophic keratitis, or dry eye disease, wherein the pharmaceutical composition includes 7,8-dihydroxyflavone or a pharmaceutically acceptable salt thereof as an active ingredient.

In some embodiments, the corneal injury may be corneal epithelial injury and/or corneal nerve injury.

In some embodiments, the corneal injury may be a corneal chemical burn, a corneal mechanical injury, or the corneal injury caused by a related disease.

The corneal chemical burn may include corneal alkali burn, corneal acidic burn, corneal thermal burn, and the like. The corneal alkali burn may be caused by inorganic alkalis such as sodium hydroxide, potassium hydroxide, or calcium hydroxide, or by organic bases. The corneal acid burn may be caused by inorganic acids such as hydrochloric acid, sulfuric acid, nitric acid, and phosphoric acid, or by organic acids.

The corneal mechanical injury may include corneal epithelial abrasion, corneal foreign body injury, and corneal injury resulting from ophthalmic surgery. The ophthalmic surgery refers to any eyeball cutting operation, typically including cataract surgery, glaucoma surgery, retinal surgery, LASIK surgery, LASEK surgery, or corneal transplantation. The corneal epithelial abrasion may be corneal epithelial injury caused by wearing contact lenses or corneal blunt trauma.

The aforementioned related diseases that cause corneal injury may be ophthalmic diseases such as tear film dysfunction, corneal infection, corneal inflammation, ciliary nerve injury, injury to the ophthalmic branch of the trigeminal nerve, neurotrophic keratitis, and dry eye disease.

In some embodiments, neurotrophic keratitis may be caused by corneal injury. Generally, neurotrophic keratitis is a disease based on corneal nerve abnormality accompanied by diminished neural sensation and secretory function, which further impairs corneal epithelium and compromises the integrity of the tear film, epithelium and stroma. Specifically, the pathogenesis of neurotrophic keratitis is mainly due to corneal nerve injury leading to loss of corneal sensitivity. After corneal denervation, a series of corneal problems may occur, including reduced lacrimal secretion, decreased mitosis of corneal epithelial cells, lowered glycogen content in corneal epithelial cells, and shedding of microvilli on superficial epithelial cells. Such changes trigger activation of corneal inflammatory response and consequently give rise to a series of pathological changes such as corneal epithelial defects, delayed healing, and corneal ulcers.

**In** some embodiments, dry eye disease can be caused by corneal injury. **In** some cases, structural changes in the cornea following corneal injury may impair the lubricating and adhesive functions of tear mucin on the ocular surface epithelium, impair tear dynamics, and reduce tear secretion volume, thereby decreasing tear film stability and ultimately leading to dry eye disease. Corneal nerve injury caused by various factors may result in diminished corneal surface sensation, reduced blink frequency, and decreased tear secretion, thereby inducing dry eye disease. **In** other cases, corneal injury leads to keratitis, which may ultimately cause dry eye disease through multiple mechanisms such as reducing tear secretion, destabilizing the tear film, exacerbating inflammatory responses, promoting cell apoptosis, and interfering with neurophysiological mechanisms.

In other embodiments, 7,8-dihydroxyflavone and pharmaceutically acceptable salts thereof have also played a positive role in the treatment of dry eye disease caused by tear film injury and/or keratitis.

Since corneal nerves are distributed within the cornea, corneal epithelial injury is usually accompanied by corneal nerve injury.

The 7,8-dihydroxyflavone and pharmaceutically acceptable salts thereof disclosed herein may include all possible solvates, hydrates, racemates, or stereoisomers.

In some embodiments, pharmaceutically acceptable salts may be salts that retain the biological effects and properties of 7,8-dihydroxyflavone and generally have no biological or other disadvantages. The pharmaceutically acceptable salts may include acid addition salts and base addition salts. The acid addition salts may be formed with inorganic and organic acids. The base addition salts that are pharmaceutically acceptable and may have inorganic or organic counterions may be formed with inorganic or organic bases.

When formulating pharmaceutical compositions, commonly used pharmaceutically acceptable excipients such as fillers, bulking agents, binders, wetting agents, disintegrants, surfactants, and diluents are employed.

As used herein, the term "pharmaceutically acceptable excipient" means a pharmaceutically acceptable material, mixture, or vehicle relevant to the consistency of the dosage form or pharmaceutical composition. Each excipient must be compatible with the other components of the pharmaceutical composition when mixed to avoid interactions that would significantly reduce the efficacy of the active pharmaceutical ingredient disclosed herein upon administration to a patient or result in a pharmaceutical composition that is not pharmaceutically acceptable. Furthermore, each excipient must be pharmaceutically acceptable; for example, possessing a sufficiently high purity.

Suitable excipients vary depending on the specific dosage form selected. Furthermore, excipients may be selected based on their specific function within the composition. For example, certain excipients may be selected to aid in producing a uniform dosage form, certain excipients may be selected to aid in producing a stable dosage form, certain excipients may be selected to aid in carrying or delivering the active ingredient disclosed herein from one organ or part of the body to another organ or part upon administration to a patient, and certain excipients may be selected that enhance patient compliance.

The active ingredients disclosed herein may be administered systemically or locally to the eye (e.g., topical administration, intraocular administration, or administration via implantation). The active ingredient may be incorporated into a topical ophthalmic formulation for administration to the eye. The active ingredient may be combined with ophthalmically acceptable preservatives, surfactants, viscosity-increasing agents, penetration enhancers, buffers, sodium chloride, and water to form a sterile aqueous ophthalmic suspension or solution. Ophthalmic solutions can be prepared by dissolving the active ingredient in a physiologically acceptable isotonic aqueous buffer. Isotonic agents are used to adjust the isotonicity of the eye drops and are typically selected from sodium chloride or potassium chloride. Buffers serve to regulate the acidity or alkalinity of the eye drops. Buffers commonly used in the preparation of eye drops include aminocaproic acid, disodium hydrogen phosphate, and sodium dihydrogen phosphate. Stabilizers serve to stabilize the eye drop, and sodium edetate and/or sodium perborate may be used as stabilizers. pH adjusters adjust the pH of the eye drop composition, illustrative examples of which include hydrochloric acid and/or sodium hydroxide. In addition, the ophthalmic solution may contain an ophthalmical surfactant to aid in the dissolution of the active ingredients. Furthermore, the ophthalmic solution may contain viscosity-increasing substances such as hydroxymethyl cellulose, hydroxyethyl cellulose, hydroxypropyl methylcellulose, methyl cellulose, polyvinylpyrrolidone, and the like, to improve the retention of the formulation in the conjunctival sac. Gelling agents may also be used, including, but not limited to, gellan gum and xanthan gum. To prepare sterile ophthalmic ointments, the active ingredient and preservatives may be mixed in a suitable ointment base, such as mineral oil, liquid lanolin, or white petrolatum. Sterile ophthalmic gel formulations may be prepared according to known formulations for similar ophthalmic formulations by suspending the active ingredient in a hydrophilic matrix including, for example, Carbopol-940. Preservatives and tonicity agents may be incorporated therein.

Solid formulations for oral administration include tablets, pills, powders, granules, capsules, and lozenges. These solid formulations include one or more of the compounds disclosed herein and at least one excipient, such as starch or calcium carbonate. These formulations may be prepared by mixing calcium carbonate, sucrose, lactose or gelatin. In addition to basic excipients, lubricants such as talc may be used. Oral liquid formulations include suspensions, solutions, emulsions, or syrups. Besides conventional diluents such as water, various excipients such as wetting agents, sweeteners, flavoring agents, and preservatives may also be added.

Formulations for parenteral administration include sterile aqueous solutions, non-aqueous solutions, suspensions, emulsions, lyophilizates, and suppositories.

As used herein, the term "treating" any disease or disorder refers to all measures that can slow down, interrupt, prevent, control, or stop the progression of the disease or disorder, but does not necessarily mean that all symptoms of the disease or disorder disappear. It also includes preventive treatment of the symptoms, especially in patients who are prone to such diseases or disorders. In some embodiments, "treating" means improving the disease or disorder (i.e., the progression of the disease or at least one clinical symptom thereof is slowed, or arrested, or alleviated). In other embodiments, "treating" refers to alleviating or ameliorating at least one physical parameter, including physical parameters that may not be noticeable to the patient. In other embodiments, "treating" refers to modulating the disease or disorder physically (e.g., stabilization of observable symptoms) or physiologically (e.g., stabilization of a bodily parameter), or both. In other embodiments, "treating" refers to preventing or delaying the onset, occurrence, or worsening of a disease or disorder.

As used herein, the term "effective amount", "therapeutically effective amount" or "therapeutically effective dose" refers to the amount of the compound of the present disclosure that can trigger a biological or medical response in an individual (e.g., reducing or inhibiting enzyme or protein activity, or alleviating symptoms, alleviating disorders, slowing or delaying disease progression, or preventing disease, etc.).

The dosage of active ingredient in the disclosed compositions may vary; however, it is necessary that the amount of the active ingredient be such that a suitable dosage form is obtained. The active ingredient may be administered to patients (animals and humans) in need of such treatment in dosages that will provide optimal pharmaceutical efficacy. The selected dosage depends upon the desired therapeutic effect, on the route of administration, and on the duration of the treatment. The dose will vary from patient to patient depending upon the nature and severity of disease, the patient's weight, patient's special diets, concurrent medication, and other factors which those skilled in the art will recognize.

In some embodiments, an effective amount of the disclosed 7,8-dihydroxyflavone may vary depending on the patient's age, gender, and body weight, typically ranging from 0.1 to 1000 mg/kg body weight, such as 1 to 100 mg/kg body weight or 50 to 100 mg/kg body weight. Administration may be performed every other day or divided into 1 to 3 daily doses. However, the dosage may be increased or decreased depending on administration route, disease severity, gender, body weight, age and other factors, and such dosage does not limit the scope of the present disclosure in any way.

In some embodiments, the ophthalmic drop composition disclosed herein is applied at 1 to 3 drops per administration, 5 to 6 times daily, and the dosing frequency can be appropriately adjusted according to clinical symptoms. The specific dose level for an individual subject may vary in view of body weight, age, gender, physical condition, administration timing, dosing frequency, disease severity and other relevant factors.

The present disclosure also provides the following technical solutions:
In some embodiments, the present disclosure provides a use of 7,8-dihydroxyflavone in the manufacture of a medicament for preventing and/or treating corneal injury or corneal epithelial injury.

In some embodiments, the corneal injury or corneal epithelial injury may be selected from the group consisting of corneal chemical burn, corneal mechanical injury, corneal foreign body injury, corneal biological injury, corneal thermal burn, corneal injury following ophthalmic surgery, neurotrophic keratitis, and dry eye disease.

In some embodiments, the corneal injury is selected from corneal intraepithelial nerve terminal injury, and/or corneal subbasal nerve plexus injury, and/or corneal stromal nerve trunk injury.

In other embodiments, the present disclosure provides a pharmaceutical composition including 7,8-dihydroxyflavone and pharmaceutically acceptable excipients.

In some embodiments, the composition is formulated into a dosage form selected from ophthalmic formulations, suspensions, granules, capsules, powders, tablets, solutions, and drops.

In some embodiments, the ophthalmic formulation is selected from eye drops, ophthalmic ointments, ophthalmic gels, or injections.

Subjects to which the active pharmaceutical ingredient or pharmaceutical composition thereof disclosed herein is administered refer to animals. Generally, the animals are mammals. The subjects, for example, also refer to primates (such as humans, males or females), bovines, sheep, goats, horses, canines, cats, rabbits, rats, mice, fish, birds, and the like. In some embodiments, the subjects are primates. In other embodiments, the subjects are human beings.

For the purpose of facilitating an understanding of the principles of the present disclosure, the embodiments illustrated in the accompanying drawings are described in detail below. These descriptions are not intended to limit the scope of the present disclosure.

### Examples

Example 1: Testing the effects of 7,8-DHF on corneal epithelial injury, neurotrophic keratitis, and dry eye disease in a BAC eye drop-induced mouse model.

### Materials and Methods:

PBS refers to phosphate buffered saline.

BAC refers to benzalkonium chloride.

0.1% BAC refers to a content of BAC is 0.1 g per 100 mL.

0.1% sodium fluorescein refers to the content of sodium fluorescein is 0.1 g per 100 mL.

Normal control group: Mice received topical administration of 5 µL saline to each eye twice daily for 7 consecutive days.

Model group: Mice received topical administration of 5 µL 0.1 % BAC to each eye twice daily for 7 consecutive days.

7,8-DHF treatment group: Mice received topical administration of 5 µL 0.1% BAC to each eye twice daily for 7 consecutive days. Starting from Day 3 of 0.1% BAC administration, 7,8-DHF was administered orally twice per day at a dose of 100 mg/kg body weight with an administration volume of 10 µL/g, and the 7,8-DHF treatment lasted for 7 days.

Corneal Fluorescein Staining: Designating the first day of adiministration to the model group as Day 1. Corneal fluorescein staining was performed on Day 5 and Day 10 for the model group, treatment group, and normal control group, respectively. The staining procedure was as follows: after mice were anesthetized, 5 µL of 0.1% sodium fluorescein was instilled into each eye of the mice, the eyes were kept closed for several seconds to allow the sodium fluorescein to distribute as evenly as possible across the cornea, and then observed under a slit lamp. The cornea was divided into four regions, and scores were recorded for each region. The scores for each region were added to obtain the final score; the more severe the corneal injury, the higher the score. The scoring criteria were as follows:
Score 0: No fluorescein staining
Score 1: Mild punctate staining with fewer than 30 stained spots
Score 2: Punctate staining with more than 30 stained spots
Score 3: Severe diffuse but no distinct patchy staining
Score 4: Distinct patchy staining.

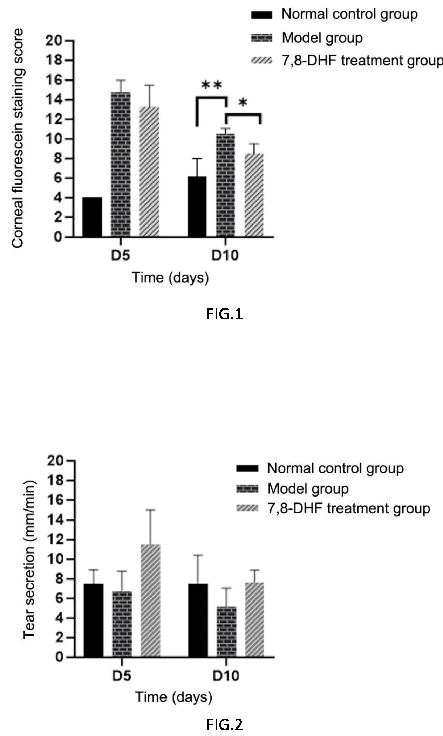

The statistical results of corneal fluorescein staining scores are shown in FIG. 1.

Conclusion: Compared with the model group, the corneal fluorescein staining scores in the 7,8-DHF treatment group were significantly lower, while the normal control group had the lowest corneal fluorescein staining scores. These results demonstrate that 7,8-DHF significantly promotes the repair of corneal epithelial injury in the BAC eye drop-induced mouse model.

Tear testing method: Designating the first day of adiministation to the model group as Day 1. Tear tests were performed on Days 5 and Day 10 for the model group, treatment group, and normal control group, respectively. The testing method was as follows: After being anesthesiad, the mouse lower eyelid was gently pulled open, and a phenol red thread was inserted into the outer third of the eyelid using forceps. Timing began immediately, and after 1 minute, the length of the reddened cotton thread was measured and recorded. The statistical results are shown in FIG. 2.

Conclusion: On the second day after the start of 7,8-DHF treatment, tear volume in the treatment group increased significantly compared to the model group. After 7 days of 7,8-DHF treatment, tear volume in the treatment group was comparable to that in the normal control group. These findings indicate that 7,8-DHF promotes the recovery of tear film function in the BAC eye drop-induced mouse model.

Tear deficiency or kinetic abnormalities lead to impaired tear film function, which can cause corneal epithelial injury and/or corneal nerve injury. Such injuries disrupt the interaction between the corneal epithelium and corneal nerves, resulting in abnormal corneal sensitivity, compromised corneal structural integrity, and corneal inflammatory responses, which in turn lead to dry eye disease and neurotrophic keratitis.In the BAC eye drop-induced mouse model, compared to the model group, the 7,8-DHF treatment group displayed markedly alleviated corneal injury and restored tear film function, demonstrating that 7,8-DHF is effective in treating dry eye disease and neurotrophic keratitis.

Example 2: Testing the effects of 7,8-DHF on corneal nerve protection and promotion of corneal nerve growth in a mouse corneal alkali burn model

### Materials and Methods:

A. Preparation of an alkali burn solution: 0.4 g of sodium hydroxide powder was weighed and dissolved in 10 mL of deionized water.
B. Mouse corneal alkali burn model: Filter paper discs with a diameter of 2 mm were prepared. After the mice were anesthesiad, 1 µL of the alkali solution was applied onto the filter paper discs. Once the filter paper discs were completely soaked with the alkali solution, they were placed onto the center of the cornea. After 8 seconds, the filter paper discs were immediately removed, and the eyeballs were rinsed with 10 mL of PBS.

7,8-DHF Treatment: Administration was initiated after modeling, once daily, at a dose of 50 mg/kg. After 7 days of administration, the mice were subjected to cardiac perfusion and tissue collection. The entire cornea was harvested to prepare flat wholemounts, which were then imaged under a fluorescence microscope.

**In** Example 2 of the present disclosure, transgenic mice were used. The corneal nerves of the transgenic mice specifically express green fluorescent protein, and thus the distribution of corneal nerves can be marked by green fluorescence signals. The anatomical characteristics of corneal nerves in the corneal epithelium, subepithelial stroma, and corneal stroma are as follows: corneal nerves originate from the ophthalmic branch of the trigeminal nerve, pass via the ciliary nerves to arrive at the cornea, run through the stromal layer, and turn by 90 degrees to form a nerve plexus in the subepithelial stroma. The plexus then gives off small vertical branches that form dense nerve terminals within the corneal epithelium. Therefore, the primary distribution features of corneal nerves are: the corneal stromal nerve trunks, the subbasal plexus, and the intraepithelial nerve terminals.

One week after alkali burn induced by sodium hydroxide at the central corneal region of mice, the distribution of corneal nerves was very sparse at the levels of the corneal stromal nerve trunks, subbasal nerve plexus, and intraepithelial nerve terminals, as shown in panels (A) to (F) of FIG. 3. After the alkali-burned mice received one week of 7,8-DHF treatment, compared to the model group, the density of corneal nerve distribution was significantly increased at the levels of the corneal stromal nerve trunks, subbasal nerve plexus, and intraepithelial nerve terminals, as shown in panels (G) to (L) of FIG. 3.

Images were acquired using a fluorescence microscope. After corneal nerve density was analyzed and quantified using the image analysis software Image J, intergroup statistical comparison was performed. Following one week of 7,8-DHF administration in alkali-burned mice, corneal nerve density was significantly higher than that of the model group, and the statistical results are presented in FIG. 5.

Conclusion: In the mouse corneal injury model established via alkali burn, 7,8-DHF can significantly increase corneal nerve density and promote corneal nerve growth. Corneal nerves function to nourish corneal epithelial cells and play a crucial role in maintaining the integrity of the corneal epithelium and promoting the proliferation and repair of corneal epithelial cells. These findings indicate that 7,8-DHF is highly effective in treating corneal injury caused by chemical burns.

In the mouse corneal alkali burn model, injuries to the corneal epithelium and corneal nerves were simulated. Corneal epithelial injury and corneal nerve injury are the primary causes of dry eye disease, while corneal nerve injury is a primary cause of neurotrophic keratitis. Example 2 demonstrates that 7,8-DHF achieves unexpected therapeutic effects in treating neurotrophic keratitis and dry eye disease induced by corneal epithelial injury and/or corneal nerve injury.

Example 3: Testing the effects of 7,8-DHF on corneal nerve protection and promotion of corneal nerve growth in a mouse corneal epithelial abrasion model.

### Materials and Methods:

A. Mouse corneal epithelial abrasion model: After mice were anesthetized, the corneal epithelium was removed to expose the corneal stroma using an electric cornea burr. The wound was horizontally extdented to the limbus.
B. 7,8-DHF Treatment: Administration was initiated after modeling, once daily, at a dose of 50 mg/kg. After 7 days of administration, the mice were subjected to cardiac perfusion and tissue collection. The entire cornea was harvested to prepare flat wholemounts, which were then imaged under a fluorescence microscope.

In Example 3 of the present disclosure, transgenic mice were used. The corneal nerves of the transgenic mice specifically express green fluorescent protein, and thus the distribution of corneal nerves can be visualized by green fluorescence signals. The primary distribution features of corneal nerves are: corneal stromal nerve trunks, subbasal nerve plexus, and intraepithelial nerve terminals.

One week after corneal epithelial abrasion in mice, the distribution of corneal nerves was very sparse at the levels of the corneal stromal nerve trunks, the subbasal nerve plexus, and the intraepithelial nerve terminals, as shown in panels (A) to (F) of FIG. 4. After mice with corneal epithelial abrasion received one week of 7,8-DHF treatment, compared with the model group, the density of corneal nerve distribution was significantly increased at the levels of the corneal stromal nerve trunks, subbasal nerve plexus, and intraepithelial nerve terminals, as shown in panels (G) to (L) of FIG. 4.

Images were acquired using a fluorescence microscope. After corneal nerve density was analyzed and quantified using the image analysis software Image J, intergroup statistical comparison was performed. After mice with corneal epithelial abrasion were treated with 7,8-DHF for one week, corneal nerve density was significantly higher than that of the model group; statistical results are shown in FIG. 6.

Conclusion: In a mouse corneal epithelial abrasion model, 7,8-DHF can increase corneal nerve density and promotes corneal nerve growth. Corneal nerves function to nourish corneal epithelial cells and play a crucial role in maintaining the integrity of the corneal epithelium and promoting the proliferation and repair of corneal epithelial cells. These indicate that 7,8-DHF is highly effective in treating corneal injury caused by mechanical factors.

In the mouse corneal epithelial abrasion model, injury to the mouse corneal epithelium and corneal nerves was simulated. Corneal epithelial injury and corneal nerve injury are the primary causes of dry eye disease, while corneal nerve injury is the primary cause of neurotrophic keratitis. Example 3 demonstrates that 7,8-DHF achieves unexpected therapeutic effects in treating neurotrophic keratitis and dry eye disease caused by corneal epithelial injury and/or corneal nerve injury.

The mouse models of chemical corneal burn, corneal epithelial abrasion, and BAC-induced corneal injury described in the examples can representatively simulate clinical corneal injuries, including but not limited to chemical corneal burn, mechanical corneal injury, corneal foreign body injury, biological corneal injury, thermal corneal burn, and corneal injury following ophthalmic surgery, as well as neurotrophic keratitis and dry eye disease resulting from corneal injury, or corneal injury associated with neurotrophic keratitis and dry eye disease. The aforementioned corneal injuries include corneal epithelial injury and/or corneal nerve injury.

Although various embodiments of the present disclosure have been described in detail herein, these embodiments are provided merely as non-limiting examples of the subject matter of the present application. Therefore, those skilled in the art will understand that various changes and modifications may be made to the present application without departing from the scope of the present application. Indeed, the present disclosure is not intended to be exhaustive or to limit the scope of the present application.

Furthermore, in the description of the representative embodiments, the methods and/or processes of the present application have been set forth with a specific sequence of steps. However, such methods or processes shall not be limited to the specific sequence of steps described. Other sequences of steps are possible. Accordingly, the specific sequence of steps disclosed herein shall not be construed as a limitation on the scope of the present application. Furthermore, the content of the application regarding methods and/or processes is not restricted to performance of the steps in the order set forth. Such an order may vary and still fall within the scope of the present application.

The foregoing merely illustrates specific embodiments of the present application to enable those skilled in the art to understand and practice the present application. Numerous modifications to these embodiments will be apparent to those skilled in the art, and the general principles defined herein may be implemented in other embodiments without departing from the spirit or scope of the present application. Accordingly, the present application is not to be limited to the embodiments described herein, but is entitled to the broadest scope consistent with the principles and novel features claimed herein.

## Claims

1. Use of 7,8-dihydroxyflavone or a pharmaceutically acceptable salt thereof in the manufacture of a medicament for preventing and/or treating corneal injury, neurotrophic keratitis, or dry eye disease.

2. The use according to claim 1, wherein the neurotrophic keratitis or dry eye disease is caused by corneal injury.

3. The use according to claim 1 or 2, wherein the corneal injury is corneal epithelial injury and/or corneal nerve injury.

4. The use according to claim 3, wherein the corneal nerve injury is selected from corneal intraepithelial nerve terminal injury, and/or corneal subbasal nerve plexus injury, and/or the corneal stromal nerve trunk injury.

5. The use according to any one of claims 1 to 4, wherein the corneal injury is selected from corneal chemical burn; corneal mechanical injury; corneal foreign body injury; biological corneal injury; corneal thermal burn; and
corneal injury caused by tear film dysfunction, corneal infection, corneal inflammation, ciliary nerve injury, injury to ophthalmic branch of trigeminal nerve, neurotrophic keratitis, dry eye disease, or ophthalmic surgery.

6. A pharmaceutical composition comprising 7,8-dihydroxyflavone or a pharmaceutically acceptable salt thereof and pharmaceutically acceptable excipients, wherein the pharmaceutical composition is used for preventing and/or treating corneal injury, neurotrophic keratitis, or dry eye disease.

7. The pharmaceutical composition according to claim 6, wherein the composition is an oral formulation, an injectable formulation, or an ophthalmic formulation; wherein,
optionally, the oral formulation is selected from a tablet, a capsule, a granule, a powder, an oral solution, or an oral suspension;
optionally, the injectable formulation is selected from an injection, a powder for injection, or a concentrated solution for injection;
optionally, the ophthalmic formulation is selected from a patch, a lotion, a wash, a douche, an irrigation, an eye drop, an eye ointment, an ophthalmic gel, a solution for intraocular injection, a fumigant, or an eye mask.

8. A method for preventing and/or treating corneal injury, neurotrophic keratitis, or dry eye disease, comprising administering to a subject 7,8-dihydroxyflavone or a pharmaceutically acceptable salt thereof, or the pharmaceutical composition according to any one of claim 6 or 7.
